# FASCICULE DE BREVET EUROPEEN

(11) **EP 3 530 662 B1**
(45) Date de publication et mention de la délivrance du brevet: **17.03.2021**
(21) Numéro de dépôt: 19159794.7
(22) Date de dépôt: 27.02.2019
(51) Int. Cl.: C07D 487/16

(54) **PROCÉDÉ DE PRÉPARATION D'HEPTAZINES**
HERSTELLUNGSVERFAHREN VON HEPTAZINEN
METHOD FOR PREPARING HEPTAZINES

(30) Priorité: 27.02.2018 FR 1851715
(43) Date de publication de la demande: 28.08.2019
(73) Titulaire: CENTRE NATIONAL DE LA RECHERCHE SCIENTIFIQUE, 75016 Paris (FR); Ecole Normale Supérieure de Cachan, 94235 Cachan Cedex (FR)
(72) Inventeur: GALMICHE, Laurent, 72100 Le Mans (FR); AUDEBERT, Pierre, 87000 Limoges (FR)
(74) Mandataire: Lavoix

(56) Documents cités:
- CN-A- 106 883 240
- DE-A1-102012 007 529
- VIKAS D GHULE ET AL: "Computational design and structure-property relationship studies on heptazines", JOURNAL OF MOLECULAR MODELING, SPRINGER, DE, vol. 17, no. 11, 12 février 2011 (2011-02-12), pages 2927-2937, XP019968430, ISSN: 0948-5023, DOI: 10.1007/S00894-011-0959-X
- TATYANA SAPLINOVA ET AL: "2,5,8-Trihydrazino- s -heptazine: A Precursor for Heptazine-based Iminophosphoranes", ZEITSCHRIFT F?R ANORGANISCHE UND ALLGEMEINE CHEMIE, vol. 635, no. 15, 17 septembre 2009 (2009-09-17), pages NA-NA, XP055062835, ISSN: 0044-2313, DOI: 10.1002/zaac.200900311

## Description

La présente invention a pour objet un nouveau procédé de préparation d'heptazines. Elle a également pour objet les heptazines telles qu'obtenues.

A ce jour, le seul précurseur d'heptazine possédant des groupements échangeables connu est la trichloroheptazine (Schwarzer et al., Coordination Chemistry Reviews, 2013, 257, 2032-2062 ; Kroke et al., New J. Chem., 2002, 26, 508-512). Cette molécule est très sensible à l'hydrolyse et est très peu soluble : en effet, elle doit être purifiée par sublimation ou extraction au Soxhlet au toluène. En outre, la trichloroheptazine se prépare par chauffage à 120-140°C d'un mélange intime du sel de potassium de la trihydroxyheptazine et de pentachlorure de phosphore, un réactif électrophile extrêmement agressif, et il faut régulièrement évacuer le chlorure d'hydrogène gazeux formé, qui est lui aussi un composé agressif et toxique. Il se forme aussi une certaine quantité de dichlore gazeux agressif, par décomposition (réaction parasite) du pentachlorure de phosphore, qu'il faut détruire en même temps que l'acide chlorhydrique.

Ainsi, le précurseur utilisé à ce jour présente de nombreux inconvénients, à la fois concernant son caractère très peu soluble mais aussi son procédé de préparation compliqué et coûteux.

DE 10 2012 007529 décrit des dérivés d'heptazine trisubstitués par des hétérocycles ainsi que leurs procédés de préparation à partir de melem et du phthaloyldichorure.

Il existe donc à ce jour un besoin pour un dérivé d'heptazine présentant des propriétés de solubilité améliorées. Il existe également un besoin pour un procédé simplifié permettant la préparation d'heptazines ou de ses dérivés avec un rendement satisfaisant.

La présente invention a pour but de fournir un dérivé d'heptazine très soluble.

La présente invention a également pour but de fournir un procédé de préparation d'heptazines et de ses dérivés, simple, et présentant un rendement satisfaisant.

Elle a également pour but de fournir des heptazines et des dérivés d'heptazines originaux.

Ainsi, la présente invention concerne un procédé de préparation d'un composé de formule (I) suivante : 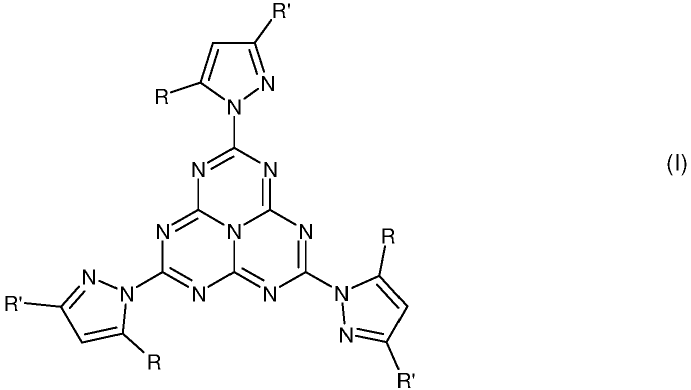 dans laquelle R et R' représentent, indépendamment l'un de l'autre, un groupe alkyle comprenant de 1 à 6 atomes de carbone ou un groupe aryle comprenant de 6 à 10 atomes de carbone ;
ledit procédé comprenant la réaction de trihydrazinoheptazine de formule (II) suivante : 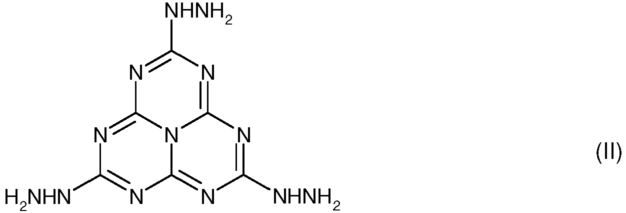 avec une cétone de formule (III) suivante : 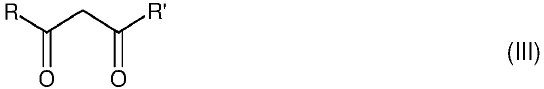 dans laquelle R et R' sont tels que définis ci-dessus.

Ce procédé, simple, permet donc d'obtenir un dérivé d'heptazine, de formule (I), très soluble et possédant des groupes facilement échangeables.

Le procédé selon l'invention permet d'obtenir des rendements satisfaisants. En particulier, ce procédé ne nécessite qu'une seule étape de chromatographie, pour un rendement moyen de 30-35% à partir de la trihydrazinoheptazine.

Selon l'invention, le terme "alkyle" désigne des groupes aliphatiques hydrocarbonés, saturés, linéaires ou ramifiés, comprenant, sauf mention contraire, de 1 à 6 atomes de carbone. A titre d'exemples, on peut citer les groupes méthyle, éthyle, n-propyle, isopropyle, butyle, isobutyle, tertbutyle ou pentyle.

Selon l'invention, les groupes aryles sont des groupes aromatiques cycliques comprenant entre 6 et 10 atomes de carbone. A titre d'exemples de groupes aryles, on peut citer les groupes phényle ou naphtyle.

Selon un mode de réalisation, dans les formules (I) et (III), les groupes R et R' sont identiques. De préférence, la cétone de formule (III) est donc une cétone symétrique.

Selon un mode de réalisation, les groupes R et R' sont des groupes alkyles comprenant de 1 à 4 atomes de carbone, et sont de préférence des groupes éthyles.

De préférence, dans les formules (I) et (III), les groupes R et R' sont identiques et sont choisis parmi les groupes alkyles comprenant de 1 à 4 atomes de carbone, notamment sont des groupes éthyles.

Préférentiellement, la cétone de formule (II) est la 3,5-heptanedione.

Selon un mode de réalisation, la trihydrazinoheptazine présente un taux d'humidité inférieur à 10%. Ce taux d'humidité peut être contrôlé par exemple par la méthode Karl-Fischer bien connue de l'homme du métier.

De préférence, la trihydrazinoheptazine présente un taux d'humidité réduit afin que ce produit de départ soit le plus sec possible.

Selon un mode de réalisation, la trihydrazinoheptazine est préalablement soumise à une étape de séchage.

Cette étape de séchage permet d'éliminer le plus possible d'eau avant la réaction de la trihydrazinoheptazine avec la cétone de formule (II).

De préférence, l'étape de séchage est effectuée pendant une durée comprise entre 12 h et 120 h, de préférence entre 24 h et 48 h.

Cette étape de séchage peut notamment être effectuée en présence d'au moins un agent desséchant.

Parmi les agents desséchants, on peut citer par exemple P₂O₅, CaO, KOH, MgO, Na₂SO₄ activé ou MgSO₄ activé. On peut citer en outre les tamis moléculaires activés ou encore les courants de gaz préalablement desséché. De préférence, cette étape est effectuée sans traitement thermique.

Selon un mode de réalisation, l'étape de séchage est précédée d'une étape de lavage de la trihydrazinoheptazine. En particulier, la trihydrazinoheptazine est lavée avec de l'eau pure, puis avec de l'éthanol puis de l'éther éthylique, notamment afin d'éliminer le plus possible d'eau.

Selon un mode de réalisation, avant la réaction avec la cétone de formule (III), la trihydrazinoheptazine est lavée, notamment avec de l'eau pure, puis avec de l'éthanol puis de l'éther éthylique, puis séchée, en particulier pendant au moins 12 heures, par exemple dans un dessiccateur contenant de l'anhydride phosphorique (P₂O₅).

Le procédé de l'invention peut comprendre en outre une étape de broyage de la trihydrazinoheptazine.

Selon un mode de réalisation, la trihydrazinoheptazine est soumise à une étape de broyage. De préférence, cette étape de broyage est effectuée après l'étape de séchage susmentionnée, et donc avant l'étape de réaction avec la cétone.

Cette étape de broyage permet de façon avantageuse de réduire la taille des particules de la trihydrazinoheptazine. De préférence, la trihydrazinoheptazine broyée est sous forme de particules dont la taille est inférieure à 100 µm.

Selon l'invention, la taille des particules correspond au diamètre moyen desdites particules.

Selon un mode de réalisation, l'étape de broyage susmentionnée consiste en une étape de broyage mécanique dans un broyeur, de préférence dans un broyeur planétaire à billes, pendant une durée comprise entre 5 minutes et 30 minutes, et de préférence entre 5 minutes et 10 minutes.

Selon un mode de réalisation avantageux, le procédé de préparation du composé de formule (I) susmentionnée comprend les étapes suivantes :
- une étape de séchage de la trihydrazinoheptazine, éventuellement précédée d'une étape de lavage telle que décrite plus haut, de préférence pendant au moins 12 heures, notamment dans un dessiccateur, par exemple avec de l'anhydride phosphorique ;
- une étape de broyage de la trihydrazinoheptazine séchée, de préférence dans un broyeur mécanique, comme décrit plus haut, et
- une étape de réaction de la trihydrazinoheptazine séchée et broyée avec la cétone de formule (III) susmentionnée.

De préférence, le procédé de l'invention est mis en œuvre avec un excès de cétone par rapport à la trihydrazinoheptazine. En particulier, le ratio entre le nombre de moles de cétone et le nombre de moles de trihydrazinoheptazine est supérieur ou égal à 1, et de préférence égal à 1,5.

Selon l'invention, l'étape de réaction entre le composé de formule (II) et le composé de formule (III) peut être effectuée en présence d'un catalyseur.

Parmi les catalyseurs, on peut par exemple citer les acides de Bronsted tels que tout acide sulfonique de formule R-SO₃H, acides carboxyliques de formule R-CO₂H, acides minéraux de formule XH (eg X = halogène, NO₃, SO₄-, HSO₄,) ainsi que les acides de Lewis de type MXₙ. A titre non limitatif, on peut notamment citer AlCl₃, MgCl₂ et FeCl₃.

De préférence, le catalyseur utilisé est l'acide toluènesulfonique.

La présente invention concerne également le composé de formule (I) susmentionné.

A titre de composé de formule (I) préféré, on peut citer les composés dans lesquels R et R' sont des groupes alkyles identiques, notamment des groupes éthyles.

Les composés de formule (I) sont particulièrement avantageux car ils permettent d'étendre la palette des groupes échangeables sur le cœur heptazine. En effet, en comparaison avec le dérivé trichloré susmentionné, le nombre de nucléophiles capables de réagir est nettement plus important, du fait de la faible solubilité, ainsi que de la forte réactivité de ce dernier.

Ainsi, les composés de formule (I) sont utilisés à titre de composés intermédiaires pour la préparation de différents dérivés d'heptazines.

La présente invention concerne donc également les composés de formule (IV) suivante : 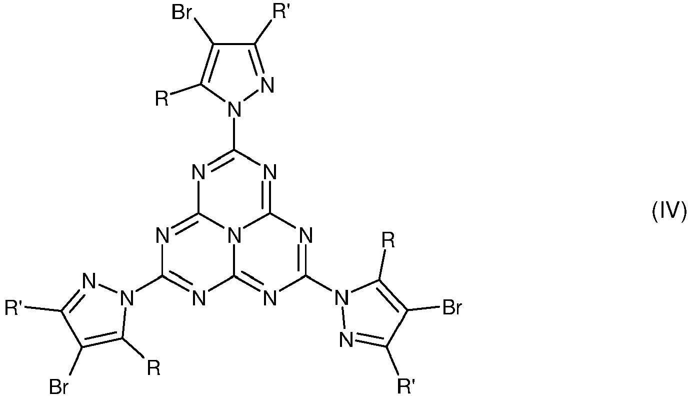 dans laquelle R et R' sont tels que définis ci-dessus.

De préférence, dans la formule (IV), R et R' sont des groupes alkyles, notamment identiques, et en particulier des groupes éthyles.

La présente invention concerne également un procédé de préparation d'un composé de formule (IV) susmentionnée, comprenant une étape de réaction du composé de formule (I) telle que définie ci-dessus avec un agent de bromation, notamment avec du dibrome.

Parmi les agents de bromation, on peut également citer le N-bromosuccinimide (NBS) et ses analogues, ou encore la dibromohydantoïne ou CBr₄/hv, etc...

Selon un mode de réalisation, cette étape de bromation est effectuée en présence d'un excès de brome, par exemple de l'ordre de 5% à 20%.

La bromation s'effectue préférentiellement dans un solvant inerte tel que le dichlorométhane, à température ambiante.

De préférence, le dibrome est ajouté en excès dans du dichlorométhane.

On peut également citer comme solvant inerte l'acétonitrile, le chloroforme, ou tout solvant de propriétés analogues.

Ensuite, l'éventuel excès de dibrome peut être éliminé par un lavage, par exemple par une solution diluée de thiosulfate de sodium dans l'eau. On peut aussi l'éliminer simplement par évaporation.

La présente invention concerne également l'utilisation du composé de formule (I) telle que définie ci-dessus, pour la mise en œuvre de réactions de substitution nucléophile.

La présente invention concerne également un procédé de préparation d'un composé de formule (V) suivante : 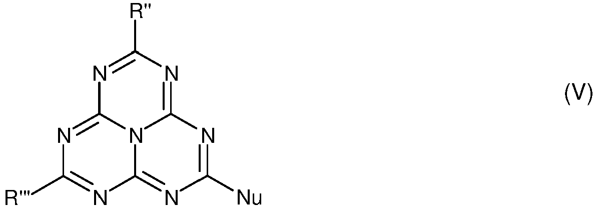 dans laquelle :
- Nu représente un groupe nucléophile,
- R" et R'", indépendamment l'un de l'autre, représentent Nu ou un groupe de formule (A) : 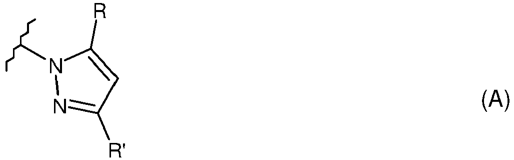 R et R' étant tels que définis ci-dessus dans la formule (I),
   ledit procédé comprenant une étape de substitution nucléophile du composé de formule (I) telle que définie ci-dessus avec un agent nucléophile.

Selon un mode de réalisation, dans la formule (V), R" et R'" sont des groupes de formule (A). Selon un autre mode de réalisation, dans la formule (V), R" est Nu et R''' est un groupe de formule (A). Selon un autre mode de réalisation, R" et R'" sont Nu.

Les composés de formule (V) sont préparés par substitution nucléophile à partir des composés de formule (I).

A titre d'agent nucléophile, on peut par exemple mentionner des amines, notamment de formule RR'NH ou RNH₂, des alcoolates de formule ROM (M = Li, Na, K, Cs), des thiols de formule RSH, des thiolates de formule RSM (M = Li, Na, K, Cs), des sélénates, des tellurates, des amidures, y compris aromatiques tels que des sels de pyrroles, imidazoles, indoles, des carbanions moyennement ou faiblement basiques (eg cériques, cuprates...), les groupes R et R' mentionnés dans ce paragraphe représentant des groupes alkyles.

Les conditions de réaction de ce procédé de substitution nucléophile sont bien connues de l'homme du métier et sont identiques à celles de toute réaction de substitution nucléophile.

Ce procédé permet d'obtenir des composés comprenant un, deux ou trois groupes Nu tels que mentionné ci-dessus.

La présente invention concerne également des composés de formule (VI) ou (VII) suivante : 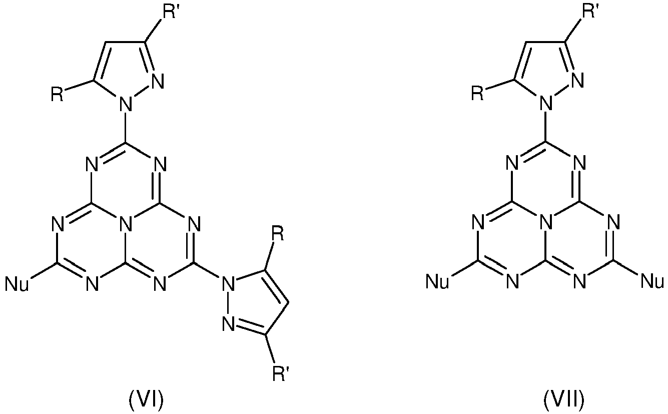 R, R' et Nu étant tels que définis ci-dessus.

De préférence, dans les formules (VI) et (VII), Nu représente un groupe amino (-NH₂) ou aminoalkyle (-NHAlk ou -N(Alk)(Alk')), ou un groupe hétérocycloalkyle comprenant au moins un atome d'azote, par exemple de type morpholine ou pipéridine.

La présente demande décrit également un composé de formule (VIII) suivante : 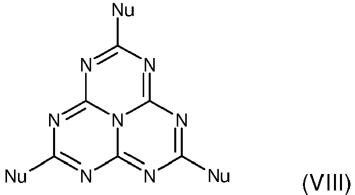 dans laquelle Nu représente un groupe nucléophile choisi parmi les amines cycliques, de préférence de formule (B) suivante : 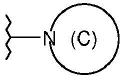 ledit cycle (C) formant avec l'atome d'azote qui le porte un hétérocycloalkyle, comprenant de 4 à 12 atomes, et comprenant éventuellement au moins un autre hétéroatome.

Selon un mode de réalisation, les composés de formule (VIII) sont différents des composés suivants :

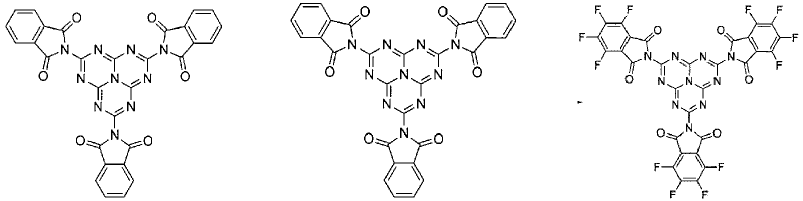

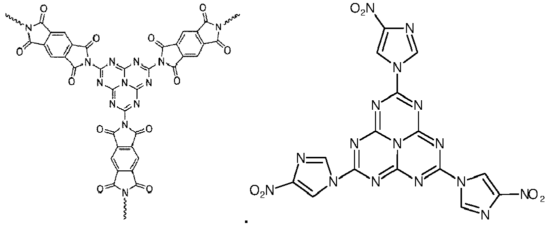

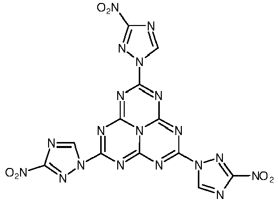

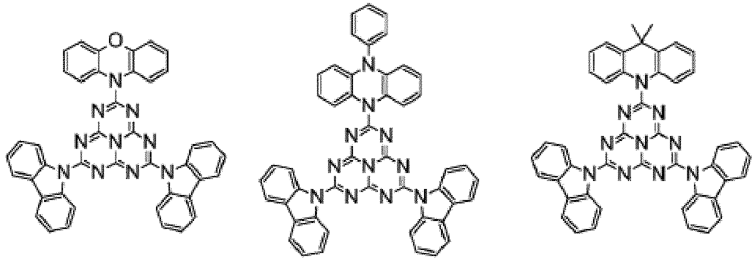

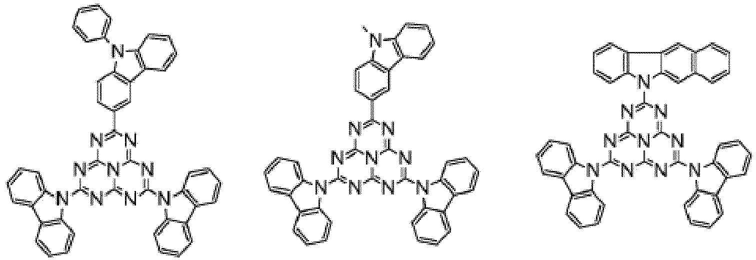

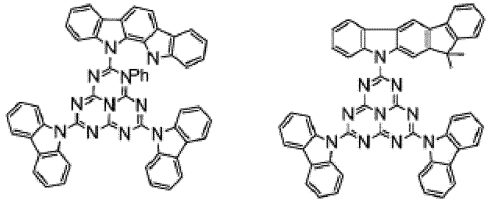

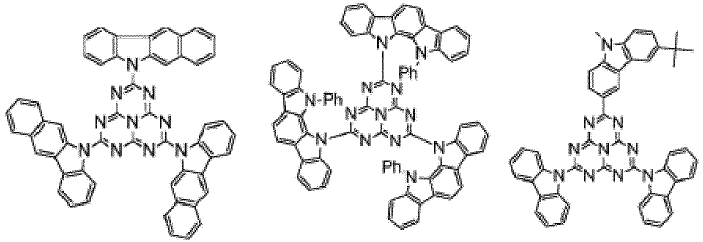

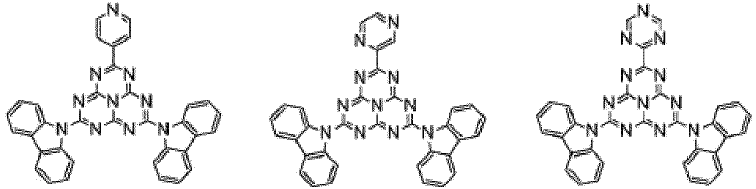

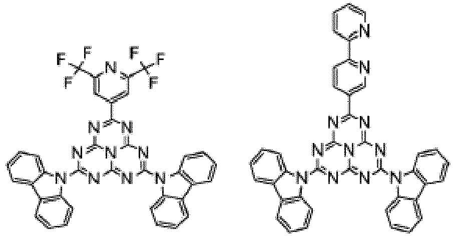

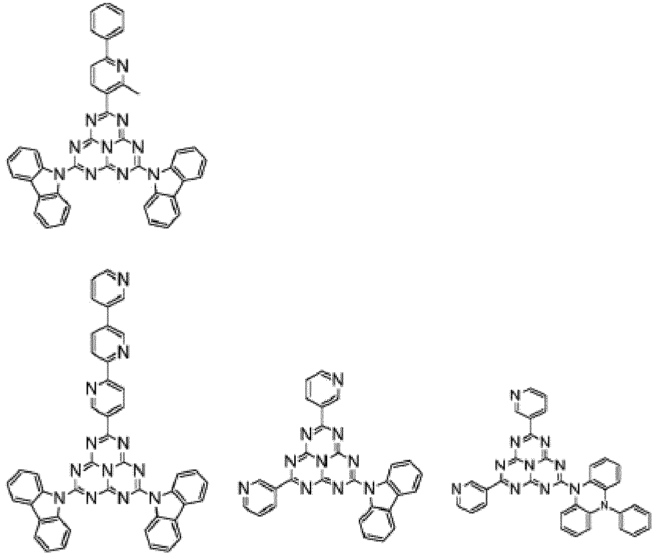

La présente demande décrit également un composé de formule (IX) suivante : 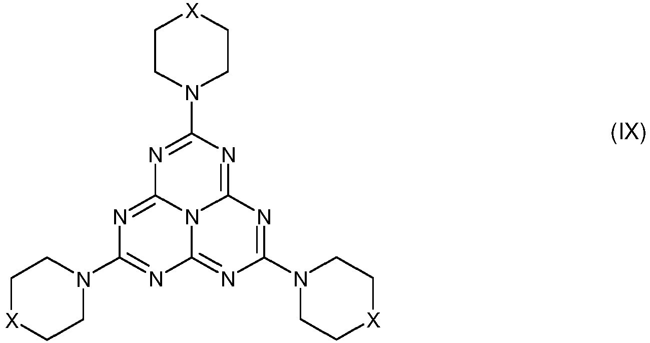 dans laquelle X représente O ou un groupe -CH(R₁), R₁ représentant H ou un groupe hétérocycloalkyle comprenant au moins un atome d'azote, éventuellement protégé.

### EXEMPLES

### Exemple 1 : Préparation de la 2,5,8-tris(3,5-diéthylpyrazol-1-yl)-heptazine

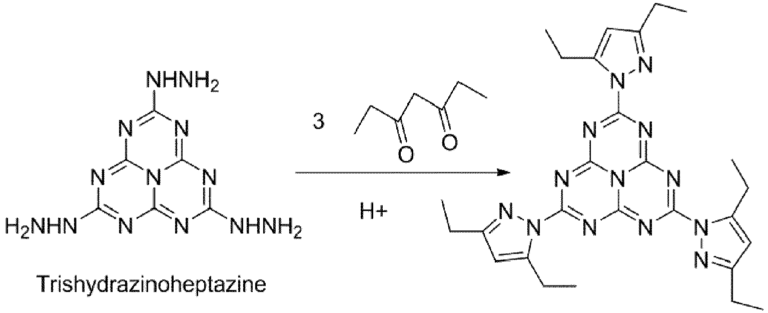

La trihydrazinoheptazine (1 g soit 3,8.10⁻³ mol) est introduite dans un broyeur planétaire d'agate (broyeur Fritsch, de type "Pulvérizette") équipé d'un bol de broyage de 80 ml, avec 10 billes d'agate d'un diamètre de 0,5 cm (même fournisseur), et on ajoute 4,5 eq. de 3,5-heptanedione (Sigma-Aldrich) (correspond à 1,5 fois la stœchiométrie nécessaire), ainsi que 0,5 g d'acide toluènesulfonique (Prolabo) comme catalyseur. Le bol de broyage est alors introduit dans le broyeur, et laissé tourner 8 mn à une vitesse de 500 tours/mn.

Après le broyage, le contenu du bol est alors repris dans le minimum de dichlorométhane (trois fois 50 ml environ), avant d'être chromatographié (éluant éther de pétrole (EP) 80% - acétate d'éthyle (EtOAc) 20%, en augmentant progressivement la quantité d'acétate d'éthyle jusqu'à 100%) pour obtenir 700 mg de 2,5,8-tris(3,5-diéthylpyrazol-1-yl)-heptazine pure (rendement d'environ 35%).

### Exemple 2 : Préparation de la 2,5,8-tris(3,5-diéthyl-4-bromo-pyrazol-1-yl)-heptazine

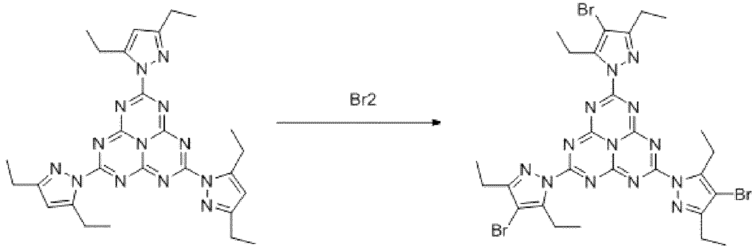

Pour la réaction de bromation de la 2,5,8-tris(3,5-diéthylpyrazol-1-yl)-heptazine, on utilise simplement un léger excès de dibrome dans du dichlorométhane (DCM)(voir Schéma) à température ambiante.

A 540 mg (10⁻³ mol) dissous dans 20 ml de DCM, on ajoute 600 mg de dibrome dissous dans 10 ml de DCM. On laisse agiter pendant une heure à température ambiante. Après la réaction, l'excès de dibrome est éliminé par un lavage par une solution diluée de thiosulfate de sodium dans l'eau. La solution est alors séchée et évaporée sous vide. Une chromatographie dans les mêmes conditions que la molécule de départ fournit le composé tribromé (580 mg, rendement 74%).

### Exemple 3 : Préparation de la 2,5,8-tris(N-morpholino)-heptazine

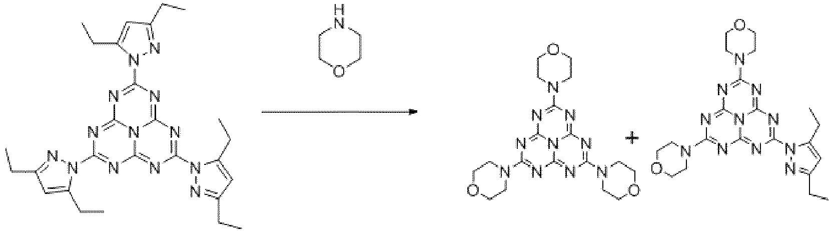

A 0,54 g, (10⁻³ mol) de 2,5,8-tris(3,5-diéthylpyrazol-1-yl)-heptazine dissoute dans 50 ml d'acétonitrile, on ajoute 4 équivalents de morpholine (Sigma-Aldrich) (350 mg, préalablement en solution dans l'acétonitrile, soit un excès de l'ordre de 1,3 par rapport à la stœchiométrie), on laisse agiter pendant 1/4h puis on porte à reflux pendant une heure. Dans ces conditions uniquement le dérivé trisubstitué est formé. On rajoute 5g de silice environ dans la solution, qui est ensuite évaporée de manière à effectuer un dépôt solide, puis le produit final est purifié par chromatographie, (éluant 50% EP/EtOAc → 100% EtOAc) pour obtenir 300 mg de Tris(N-morpholino)heptazine pure (rendement 58%).

Si l'on agite en chauffant à environ 40°C, on forme un peu de produit de di-substitution (voir Schéma) comme sous-produit. On obtient alors environ 40% de tris(morpholino)heptazine et 15% de bis(N-morpholino)-(3,5-diéthylpyrazol-1-yl)-heptazine.

Les mêmes conditions que celles de l'exemple 3 ci-dessus peuvent être appliquées pour préparer d'autres dérivés d'heptazine, en remplaçant la morpholine par de la pipéridine, de la pyrrolidine ou de la 2-éthylhexylamine.

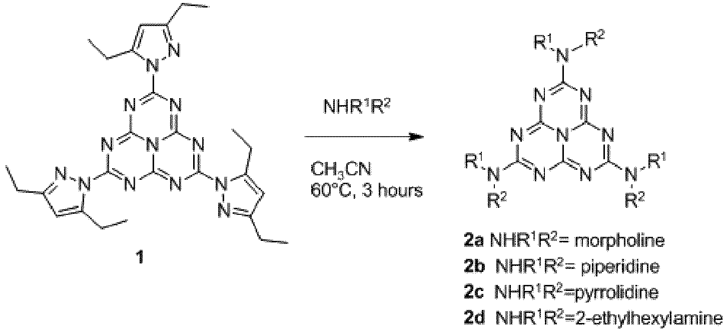

Il est également possible, à partir de 2,5,8-tris(3,5-diéthylpyrazol-1-yl)-heptazine, de préparer d'autres dérivés d'heptazine selon le schéma réactionnel ci-dessous :

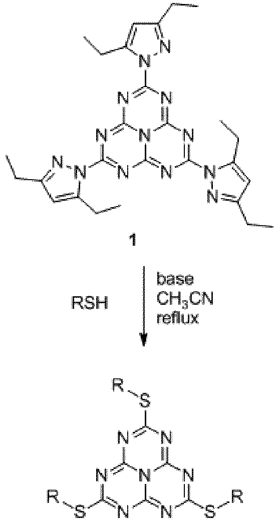

### Exemple 4 : Préparation de la 2,5,8-tris(1-^{t}butylcarboxy-4,4'-bispiperidin-1'-yl)-heptazine

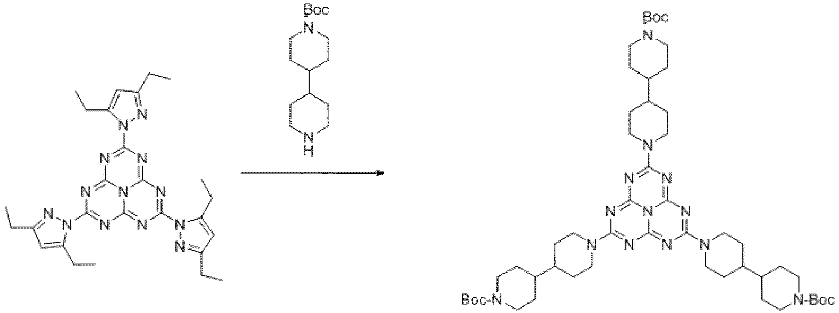

A 0,54 g (10⁻³ mol) de 2,5,8-tris(3,5-diéthylpyrazol-1-yl)-heptazine dissoute dans 50 ml d'acétonitrile, on ajoute 4 équivalents d'amine (soit 1,072 g correspondant à un excès de l'ordre de 1,3 par rapport à la stœchiométrie), on laisse agiter pendant 1/4h puis on porte à reflux pendant une heure. La réaction est traitée exactement comme dans l'exemple 3 pour fournir environ 800 mg de produit pur (rendement 50%).

### Exemple 5 : Préparation de la 2,5,8-tris(1-fluoro-2-méthyl-phen-3-yl)-heptazine

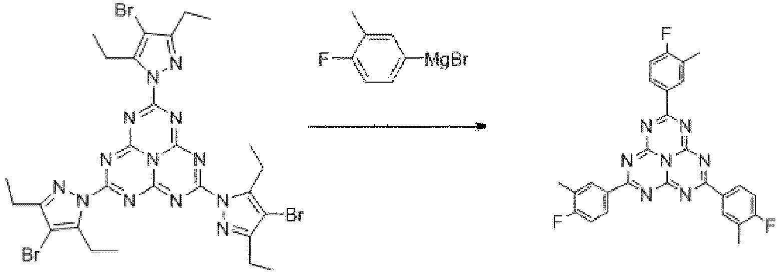

A une solution de 2,5,8-tris(3,5-diéthyl-4-bromo-pyrazol-1-yl)-heptazine refroidie à 0°C dans du THF anhydre, et sous couverture de gaz inerte (azote), on ajoute à l'aide d'une seringue 3,3 équivalents de 3-bromure de 1-fluoro-3-méthylphenylmagnésium. La solution est agitée une heure, puis laissée remonter à température ambiante. On évapore ensuite le THF, on reprend avec du DCM puis on chromatographie le produit dans les mêmes conditions que décrites par C. Adachi (Jie Li, Hiroko Nomura, Hiroshi Miyazaki and Chihaya Adachi, Chem. Commun. 2014, 50, 6174 (EP 95% AcOEt 5%) pour obtenir le produit pur, de couleur vert pâle et fluorescent dans le jaune, avec un rendement de l'ordre de 30%.

## Revendications

1. Procédé de préparation d'un composé de formule (I) suivante : 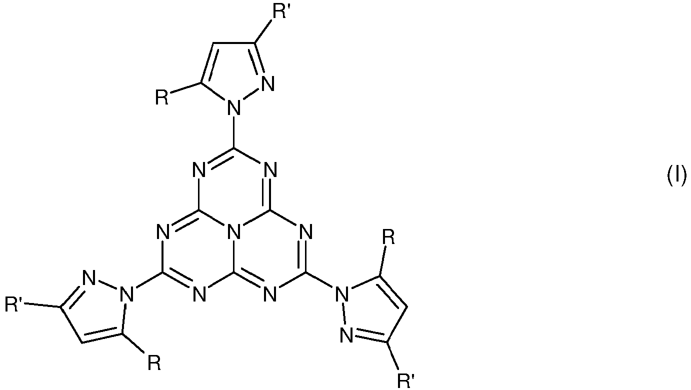 dans laquelle R et R' représentent, indépendamment l'un de l'autre, un groupe alkyle comprenant de 1 à 6 atomes de carbone ou un groupe aryle comprenant de 6 à 10 atomes de carbone ;
ledit procédé comprenant la réaction de trihydrazinoheptazine de formule (II) suivante : 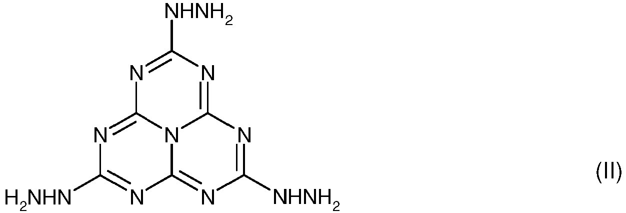 avec une cétone de formule (III) suivante : 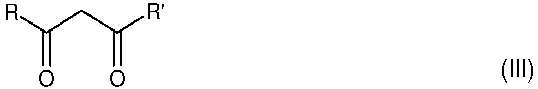 dans laquelle R et R' sont tels que définis ci-dessus.

2. Procédé selon la revendication 1, dans lequel les groupes R et R' sont identiques et choisis parmi les groupes alkyles comprenant de 1 à 4 atomes de carbone, et sont de préférence des groupes éthyles.

3. Procédé selon la revendication 1 ou 2, dans lequel la trihydrazinoheptazine est préalablement soumise à une étape de séchage.

4. Procédé selon l'une quelconque des revendications 1 à 3, dans lequel la trihydrazinoheptazine est soumise à une étape de broyage.

5. Procédé selon l'une quelconque des revendications 1 à 4, dans lequel le ratio entre le nombre de moles de cétone et le nombre de moles de trihydrazinoheptazine est supérieur ou égal à 1, et de préférence égal à 1,5.

6. Composé de formule (I) : 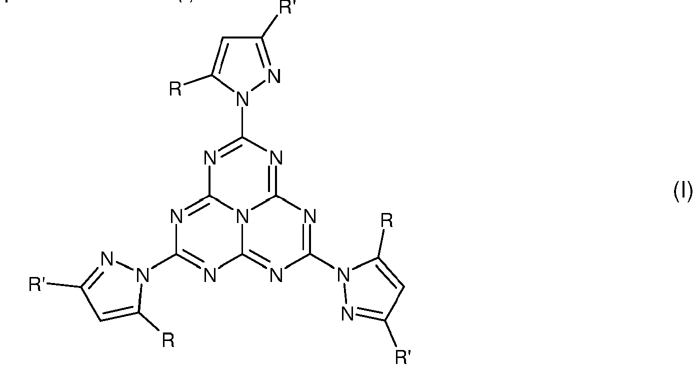 dans laquelle R et R' représentent, indépendamment l'un de l'autre, un groupe alkyle comprenant de 1 à 6 atomes de carbone ou un groupe aryle comprenant de 6 à 10 atomes de carbone.

7. Composé de formule (IV) suivante : 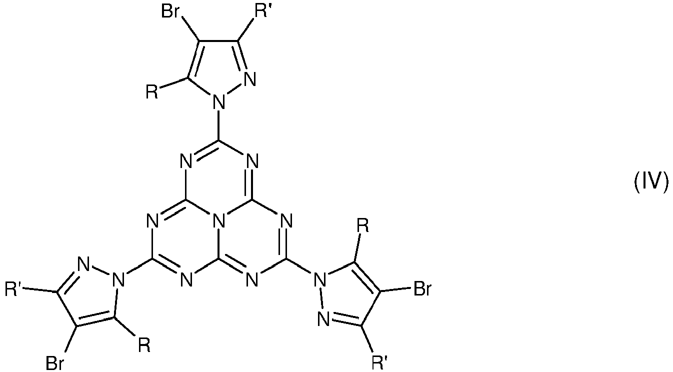 dans laquelle R et R' sont tels que définis dans la revendication 1.

8. Procédé de préparation d'un composé de formule (IV) selon la revendication 7, comprenant une étape de réaction du composé de formule (I) selon la revendication 6 avec un agent de bromation, notamment avec du dibrome.

9. Utilisation du composé de formule (I) selon la revendication 6, pour la mise en œuvre de réactions de substitution nucléophile.

10. Procédé de préparation d'un composé de formule (V) suivante : 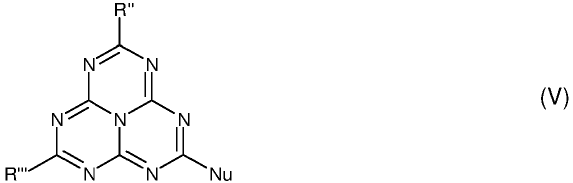 dans laquelle :
- Nu représente un groupe nucléophile,
- R" et R'", indépendamment l'un de l'autre, représentent Nu ou un groupe de formule (A) : 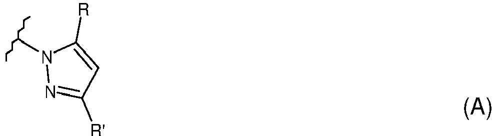 R et R' étant tels que définis dans la revendication 1,
ledit procédé comprenant une étape de substitution nucléophile du composé de formule (I) selon la revendication 6 avec un agent nucléophile.

11. Composé de formule (VI) ou (VII) suivante : 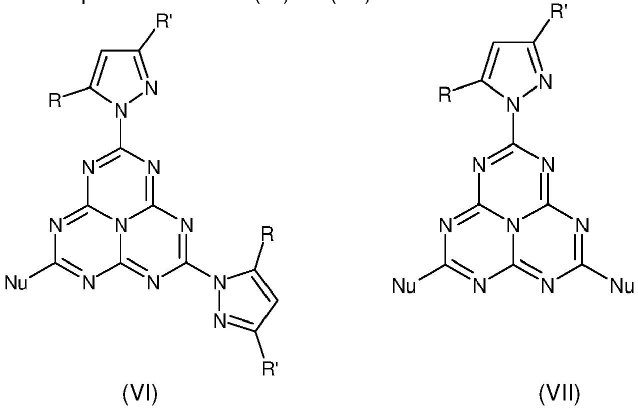 dans lesquelles :
- R et R' sont tels que définis dans la revendication 1 ; et
- Nu représente un groupe nucléophile.

## Patentansprüche

1. Verfahren zum Herstellen einer Verbindung der nachstehenden Formel (I): 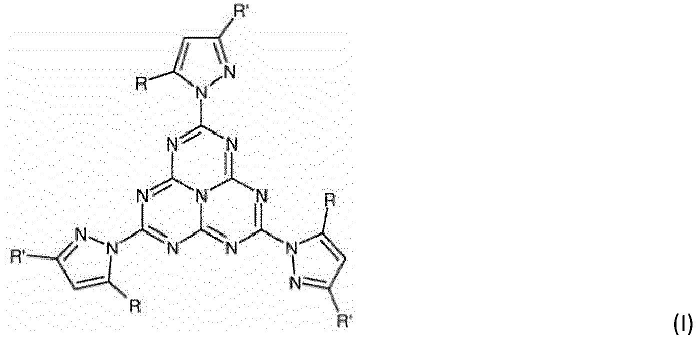 wobei R und R' unabhängig voneinander eine Alkylgruppe mit 1 bis 6 Kohlenstoffatomen oder eine Arylgruppe mit 6 bis 10 Kohlenstoffatomen darstellt,
wobei das Verfahren die Reaktion von Tryhydrazinoheptazin der nachstehenden Formel (II): 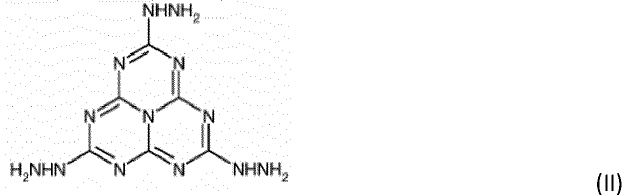 mit einem Keton der nachstehenden Formel (III): 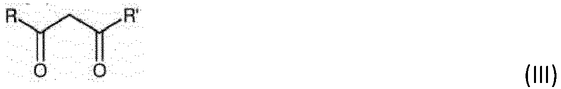 aufweist, wobei R und R' wie oben definiert sind.

2. Verfahren gemäß Anspruch 1, wobei die Gruppen R und R' gleich sind und ausgewählt sind aus den Alkylgruppen mit 1 bis 4 Kohlenstoffatomen, vorzugsweise Ethylgruppen.

3. Verfahren gemäß Anspruch 1 oder 2, wobei das Trihydrazinoheptazin vorzugsweise einem Trocknungsschritt unterzogen wird.

4. Verfahren gemäß irgendeinem der Ansprüche 1 bis 3, wobei das Trihydrazinoheptazin einem Zerkleinerungsschritt unterzogen wird.

5. Verfahren gemäß irgendeinem der Ansprüche 1 bis 4, wobei das Verhältnis zwischen der Molanzahl des Ketons und der Molanzahl des Trihydrazinoheptazins größer oder gleich 1 ist, vorzugsweise gleich 1,5.

6. Verbindung der Formel (I): 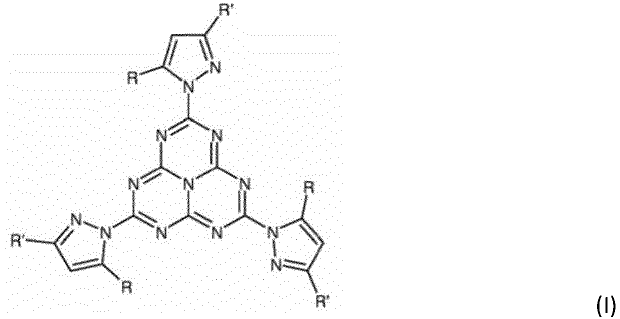 wobei R und R' unabhängig voneinander eine Alkylgruppe mit 1 bis 6 Kohlenstoffatomen oder eine Arylgruppe mit 6 bis 10 Kohlenstoffatomen darstellen.

7. Verbindung der nachstehenden Formel (IV): 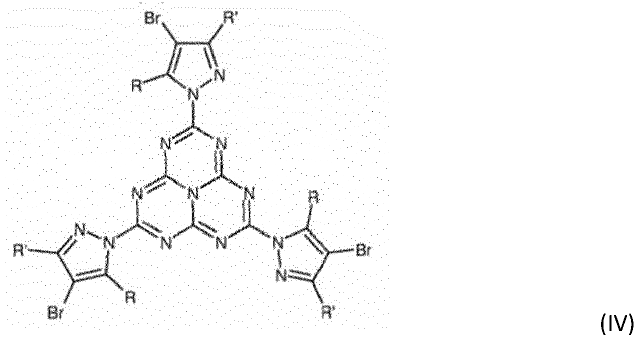 wobei R und R' wie in Anspruch 1 definiert sind.

8. Verfahren zum Herstellen einer Verbindung der Formel (IV) gemäß Anspruch 7, aufweisend einen Reaktionsschritt der Verbindung mit der Formel (I) gemäß Anspruch 6 mit einem Bromierungsmittel, insbesondere mit Brom.

9. Verwendung der Verbindung der Formel (I) gemäß Anspruch 6 zum Realisieren von nucleophilen Substitutionsreaktionen.

10. Verfahren zum Herstellen einer Verbindung der nachstehenden Formel (V): 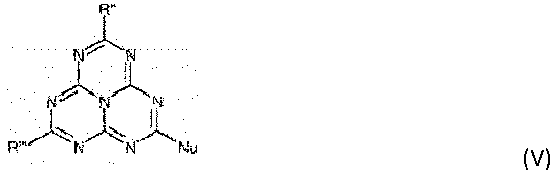 wobei:
- Nu eine nucleophile Gruppe darstellt,
- R" und R''' unabhängig voneinander Nu oder eine Gruppe der Formel (A) darstellen: 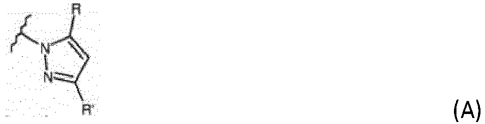 wobei R und R' wie in Anspruch 1 definiert sind,
wobei das Verfahren einen nucleophilen Substitutionsschritt der Verbindung der Formel (I) gemäß Anspruch 6 mit einem nucleophilen Mittel aufweist.

11. Verbindung der nachstehenden Formel (VI) oder (VII): 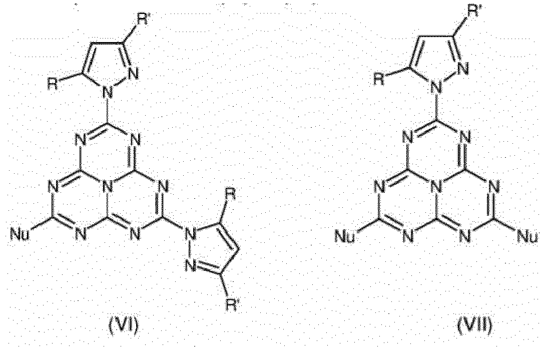 wobei:
- R und R' wie in Anspruch 1 definiert sind und
- Nu eine nucleophile Gruppe darstellt.

## Claims

1. Method of preparing a compound of the following formula (I): 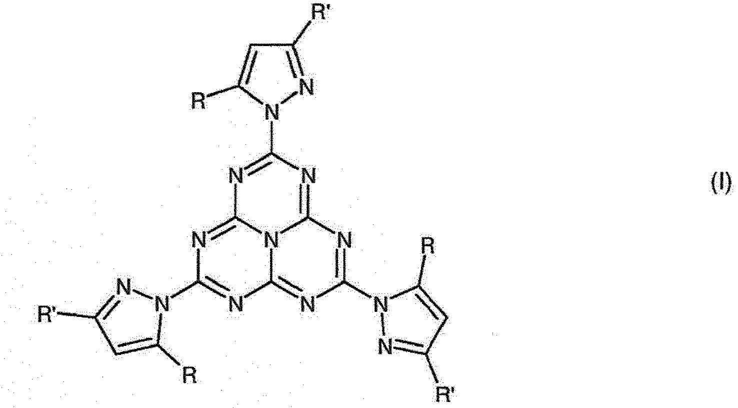 in which R and R' represent, independently of each other, an alkyl group comprising from 1 to 6 carbon atoms or an aryl group comprising from 6 to 10 carbon atoms;
said method comprising the reaction of trihydrazinoheptazine of the following formula (II): 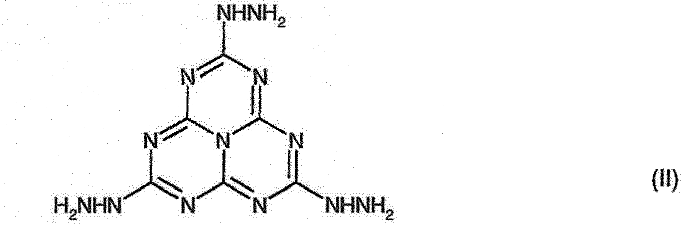 with a ketone of the following formula (III): 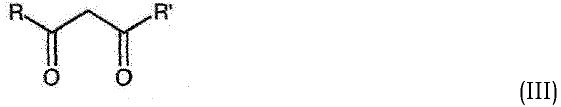 in which R and R' are as defined above.

2. Method according to claim 1, in which the groups R and R' are identical and chosen from the alkyl groups comprising from 1 to 4 carbon atoms, and are preferably ethyl groups.

3. Method according to claim 1 or 2, in which the trihydrazinoheptazine is subjected in advance to a drying step.

4. Method according to any of claims 1 to 3, in which the trihydrazinoheptazine is subjected to a grinding step.

5. Method according to any of claims 1 to 4, in which the ratio between the number of mols of ketone and the number of mols of trihydrazinoheptazine is greater than or equal to 1, and preferably equal to 1.5.

6. Compound of formula (I): 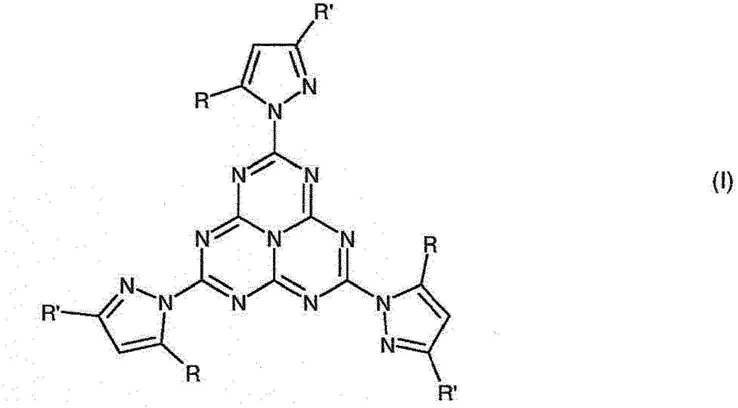 in which R and R' represent, independently of each other, an alkyl group comprising from 1 to 6 carbon atoms or an aryl group comprising from 6 to 10 carbon atoms.

7. Compound of the following formula (IV): 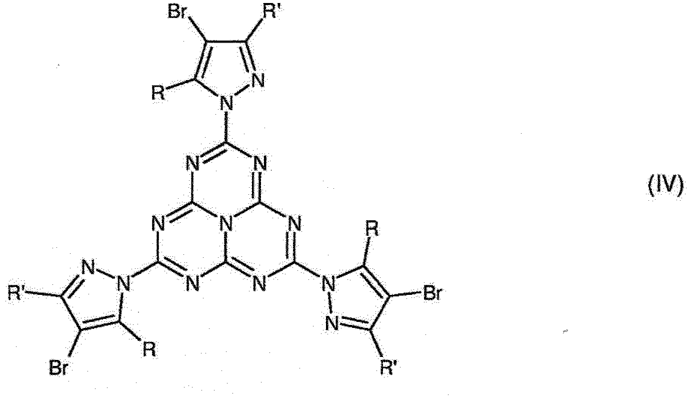 in which R and R' are as defined in claim 1.

8. Method of preparing a compound of formula (IV) according to claim 7, comprising a step of reaction of the compound of formula (I) according to claim 6 with a bromination agent, in particular with dibromine.

9. Use of the compound of formula (I) according to claim 6, for implementing nucleophilic substitution reactions.

10. Method of preparing a compound of the following formula (V): 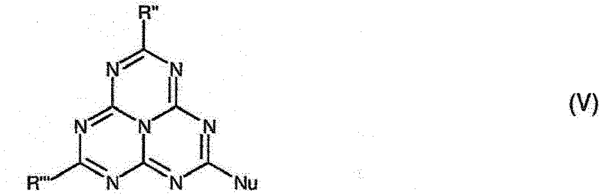 in which:
- Nu represents a nucleophilic group,
- R" and R'", independently of each other, represent Nu or a group of formula (A): 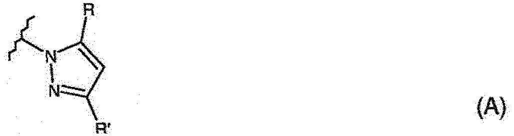 R and R' being as defined in claim 1,
said method comprising a step of nucleophilic substitution of the compound of formula (I) according to claim 6 with a nucleophilic agent.

11. Compound of the following formula (VI) or (VII): 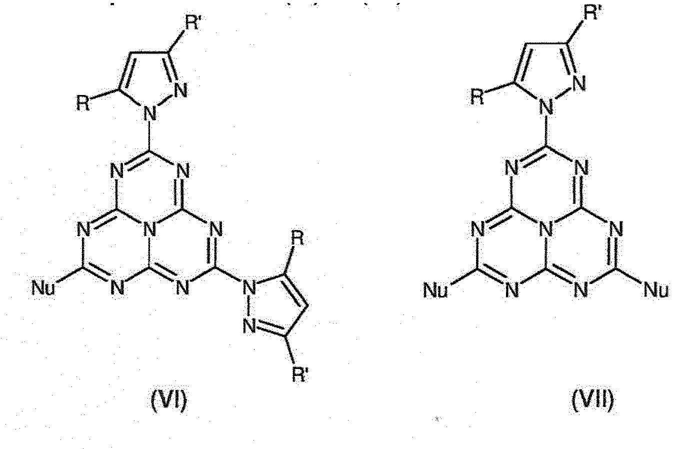 in which:
- R and R' are as defined in claim 1; and
- Nu represents a nucleophilic group.
